(19) Europäisches Patentamt
European Patent Office
Office européen des brevets

(11) Veröffentlichungsnummer: **0 088 907**
**B1**

(12) # EUROPÄISCHE PATENTSCHRIFT

(45) Veröffentlichungstag der Patentschrift: **16.10.85**

(21) Anmeldenummer: **83101601.9**

(22) Anmeldetag: **19.02.83**

(51) Int. Cl.⁴: **C 07 C 49/813,** C 07 C 49/84, C 07 C 69/76, C 07 C 45/63, C 07 C 45/46, C 07 C 67/08, C 09 K 19/12

(54) **Halogenbiphenylderivate.**

(30) Priorität: **06.03.82 DE 3208089**

(43) Veröffentlichungstag der Anmeldung: **21.09.83 Patentblatt 83/38**

(45) Bekanntmachung des Hinweises auf die Patenterteilung: **16.10.85 Patentblatt 85/42**

(84) Benannte Vertragsstaaten: **CH DE FR GB LI**

(56) Entgegenhaltungen:
**EP - A - 0 022 183**
**EP - A - 0 035 155**
**EP - A - 0 051 738**
**FR - A - 2 386 594**
**GB - A - 2 080 561**
**US - A - 4 029 594**

**MOLECULAR CRYSTALS AND LIQUID CRYSTALS+LETTERS, Band 87, Nr. 1/2, Juni 1982, Seiten 109-135, New York, USA R. DABROWSKI et al.: "Mesomorphic properties of 4-n-pentylbiphenyl derivatives"**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Römer, Michael, Dr., Im Grossen Garten 18, D-6054 Rodgau 2 (DE)**
Erfinder: **Krause, Joachim, Dr., Samuel-Morse-Strasse 14, D-6110 Dieburg (DE)**
Erfinder: **Weber, Georg, Wilhelm-Leuschner-Strasse 38, D-6106 Erzhausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue Halogenbiphenylderivate der Formel I

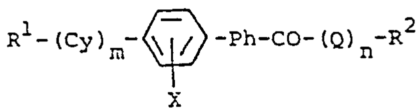
I

worin

| | |
|---|---|
| $R^1$ und $R^2$ | jeweils Alkyl oder Alkoxy mit jeweils 1-10 C-Atomen, |
| Cy | 1,4-Cyclohexylen, |
| Ph | 1,4-Phenylen, |
| Q | $-O-Cy-$ oder $-O-Ph-$ |
| m und n | jeweils 0 oder 1 und |
| X | F, Cl oder Br |

bedeuten.

Diese Substanzen können wie ähnliche, z.B. aus der DE-OS 2800553 bekannte Verbindungen als Komponenten flüssigkristalliner Dielektrika verwendet werden, insbesondere für Displays, die auf dem Prinzip der verdrillten Zelle beruhen.

Der Erfindung lag die Aufgabe zugrunde, neue flüssigkristalline Verbindungen aufzufinden, die als Komponenten flüssigkristalliner Dielektrika geeignet sind, insbesondere für nematische Phasen mit hoher optischer Anisotropie. Diese Aufgabe wurde durch die Bereitstellung der Verbindungen der Formel I gelöst.

Es wurde gefunden, dass die Verbindungen der Formel I vorzüglich als Komponenten flüssigkristalliner Dielektrika geeignet sind. Insbesondere sind mit ihrer Hilfe flüssigkristalline Phasen mit weitem nematischem Bereich, relativ hoher optischer Anisotropie und positiver dielektrischer Anisotropie herstellbar.

Die Verbindungen der Formel I sind in reinem Zustand farblos und bilden flüssigkristalline Mesophasen in einem für die elektrooptische Verwendung günstig gelegenen Temperaturbereich.

Diejenigen Verbindungen der Formel I, die optisch aktiv sind, eignen sich als chirale Dotierstoffe zur Herstellung cholesterischer Phasen wie sie auch für Farbstoff-Zellen nach White-Taylor verwendet werden können. In niedrigen Konzentrationen vermögen diese chiralen Verbindungen in der verdrillten Zelle Störungen des optischen Erscheinungsbildes durch den „umgekehrten Twist" zu verhindern (vgl. Mol. Cryst. Liq. Cryst., Band 34 (Letters), Seiten 211-217 (1977)).

Gegenstand der Erfindung sind somit die Verbindungen der Formel I sowie ein Verfahren zu ihrer Herstellung, dadurch gekennzeichnet, dass man ein Aminobiphenylderivat der Formel II

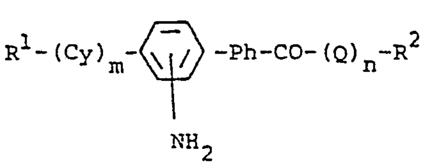
II

worin

$R^1$, $R^2$, Cy, Ph, Q, m und n die bei Formel I angegebene Bedeutung haben diazotiert und anschliessend die Diazoniumgruppe nach bekannten Methoden durch ein X-Atom ersetzt oder dass man zur Herstellung einer Verbindung der Formel I ($R^2$ = Alkyl, n = 0) ein Halogenbiphenylderivat der Formel III

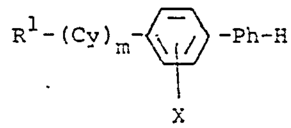
III

worin

$R^1$, Cy, Ph, m und X die bei Formel I angegebene Bedeutung haben

mit einer Carbonsäure der Formel IV

$$HOOC-R^3 \qquad IV$$

worin

$R^3$ Alkyl mit 1-10 C-Atomen bedeutet oder mit einem ihrer reaktionsfähigen Derivate in Gegenwart einer Säure oder einer Lewis-Säure umsetzt oder dass man zur Herstellung einer Verbindung der Formel I ($R^2$ = Alkoxy und/oder n = 1) eine Carbonsäure der Formel V

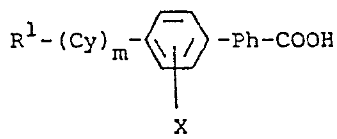
V

worin

$R^1$, Cy, Ph, m und X die bei Formel I angegebene Bedeutung haben, oder eines ihrer reaktionsfähigen Derivate mit einer Hyroxyverbindung der Formel VI

$$H-(Q)_n-R^4 \qquad VI$$

worin

$R^4$ Alkoxy mit 1-10 C-Atomen bedeutet und, falls n = 1, auch Alkyl mit 1-10 C-Atomen bedeuten kann und

Q und n die bei Formel I angegebene Bedeutung haben,

oder einem ihrer reaktionsfähigen Derivate umsetzt.

Weiterhin sind Gegenstand der Erfindung die Verwendung der Verbindungen der Formel I als Komponenten flüssigkristalliner Dielektrika, flüssigkristalline Dielektrika mit einem Gehalt an mindestens einer Verbindung der Formel I sowie elektrooptische Anzeigeelemente, die derartige Dielektrika enthalten.

Vor- und nachstehend haben $R^1$, $R^2$, Cy, Ph, Q, m, n, X, $R^3$ und $R^4$ die angegebene Bedeutung, sofern nicht ausdrücklich etwas anderes vermerkt ist.

Die Verbindungen der Formel I umfassen Ketone der Formel Ia und Ester der Formel Ib

$$R^1-(Cy)_m-PhX-Ph-CO-R^3 \qquad Ia$$
$$R^1-(Cy)_m-PhX-Ph-CO-(Q)_n-R^4 \qquad Ib$$

worin PhX einen durch ein X-Atom substituierten 1,4-Phenylenrest bedeutet.

Im einzelnen umschliesst die Formel I Ketone der Formeln Ic und Id sowie Ester der Formeln Ie bis Ij

$$R^1-PhX-Ph-CO-R^3 \qquad Ic$$
$$R^1-Cy-PhX-Ph-CO-R^3 \qquad Id$$
$$R^1-PhX-Ph-COOR^3 \qquad Ie$$
$$R^1-PhX-Ph-CO-O-Cy-R^2 \qquad If$$

$R^1-PhX-Ph-CO-O-Ph-R^2$ Ig
$R^1-Cy-PhX-Ph-COOR^3$ Ih
$R^1-Cy-PhX-Ph-CO-O-Cy-R^2$ Ii
$R^1-Cy-PhX-Ph-CO-O-Ph-R^2$ Ij

worin die Alkyl- und Alkoxygruppen jeweils 1-10, vorzugsweise 1-6, insbesondere 3, 4, 5 oder 6 C-Atome enthalten.

Die Verbindungen der Formeln Id und Ie sind bevorzugt.

Die Reste $R^1$ und $R^2$ sind vorzugsweise Alkyl.

In den Verbindungen der Formeln I sowie Ia, Ib, Id, If, Ih, Ii und Ij sind die diejenigen Stereoisomeren bevorzugt, worin die beiden Substituenten an den Cyclohexylenresten jeweils in trans-Stellung zueinander stehen.

In den Verbindungen der Formel I sowie Ia bis Ij sind die Alkyl- und Alkoxygruppen vorzugsweise geradkettig. Alkyl bedeutet vorzugsweise Methyl, Ethyl, n-Propyl, n-Butyl, n-Pentyl, n-Hexyl, ferner bevorzugt n-Heptyl, n-Octyl, n-Nonyl oder n-Decyl; Alkoxy bedeutet vorzugsweise Methoxy, Ethoxy, n-Propyloxy, n-Butyloxy, n-Pentyloxy, n-Hexyloxy, ferner bevorzugt n-Heptyloxy, n-Octyloxy, n-Nonyloxy oder n-Decyloxy.

Verbindungen der Formel I sowie Ia bis Ij mit verzweigten Alkyl- oder Alkoxygruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl), Isobutyl (= 2-Methylpropyl), 2-Methyl-butyl, Isopentyl (= 3-Methylbutyl), 2-Methylpentyl, 3-Methylpentyl, 2-Ethylhexyl, 2-Heptyl (= 1-Methylhexyl), 2-Octyl (= 1-Methylheptyl); bevorzugte verzweigte Alkoxyreste sind Isopropyloxy, 2-Methylpropyloxy, 2-Methylbutyloxy, 3-Methyl-butyloxy, 2-Methyl-pentyloxy, 3-Methyl-pentyloxy, 2-Ethyl-hexyloxy, 1-Methyl-hexyloxy, 1-Methyl-heptyloxy.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch *in situ* gebildet werden, derart, dass man sie aus dem Reaktionsgemisch nicht isoliert, sondern sofort weiter zu den Verbindungen der Formel I umsetzt.

Die Halogenbiphenylderivate der Formel I können aus den Aminen der Formel II nach den Methoden der Schiemann-Reaktion (vgl. z.B. The Merck Index, 9th Edition, Merck & Co., Inc., Rahway, N.J., USA, 1976, Seite ONR-80) oder der Sandmeyer-Reaktion (vgl. z.B. The Merck Index, l.c., Seite ONR-79) hergestellt werden.

Zweckmässig diazotiert man zunächst mit einem Salz oder einem Ester der salpetrigen Säure (wie $NaNO_2$ oder Butylnitrit) in wässerig-säurer Phase, wobei als Säure z.B. HF, HCl, HBr, $H_2SO_4$ oder $HBF_4$ verwendet werden kann, bei Temperaturen zwischen etwa −20 und +10°. Dabei kann ein zusätzliches inertes Lösungsmittel zugegen sein, z.B. ein Ether wie Tetrahydrofuran oder Dioxan oder ein Kohlenwasserstoff wie Toluol oder Xylol.

Zur Herstellung der Fluorverbindungen (I, X = F) diazotiert man zweckmässig in $HBF_4$. Dabei bilden sich die Diazonium-tetrafluorborate, die bereits bei Temperaturen zwischen etwa 10 und 100° zersetzt werden können. Diazotiert man mit $NaNO_2$ in wasserfreiem HF, so erhält man durch anschliessendes Erwärmen direkt die gewünschte Fluorverbindung.

Ein Austausch der Diazoniumgruppe gegen Cl oder Br gelingt bevorzugt in wässeriger Lösung in Gegenwart von $Cu_2Cl_2$ oder $Cu_2Br_2$ bei Temperaturen zwischen 30 und 100°.

Die Ketone der Formel Ia (= I, $R^2$ = Alkyl, n = 0) sind ferner durch Acylierung der Halogenbiphenylderivate der Formel III mit Carbonsäuren der Formel IV oder ihren reaktionsfähigen Derivaten erhältlich, zweckmässig in Gegenwart eines sauren Katalysators sowie eines inerten Lösungsmittels bei Temperaturen zwischen etwa 0° und etwa 120°. Als Derivate der Carbonsäuren der Formel IV eignen sich in erster Linie ihre Anhydride und Halogenide, z.B. die entsprechenden Säurechloride und Säurebromide. Als Katalysatoren eignen sich Säuren wie HF, $H_3PO_4$ oder Polyphosphorsäure oder, vorzugsweise, Lewis-Säuren wie $AlCl_3$, $AlBr_3$, $SnCl_4$, $ZnCl_2$, $FeCl_3$, $SbCl_5$, $BF_3$ oder dessen Etherat, als Lösungsmittel z.B. $CS_2$, Kohlenwasserstoffe wie Hexan, Nitrobenzol, oder Tetramethylensulfon.

Die Ester der Formel Ib können auch durch Veresterung der Carbonsäuren der Formel V mit Alkoholen bzw. Phenolen der Formel VI erhalten werden.

Als reaktionsfähige Derivate dieser Carbonsäuren eignen sich insbesondere die Säurehalogenide, vor allem die Chloride und Bromide, ferner die Anhydride, z.B. auch gemischte Anhydride der Formel $R^1-(Cy)_m-PhX-Ph-CO-O-COCH_3$, Azide, Ester, insbesondere Alkylester mit 1-4 C-Atomen in der Alkylgruppe.

Als reaktionsfähige Derivate der Alkohole bzw. Phenole der Formel VI kommen insbesondere die entsprechenden Metall-alkoholate bzw. -phenolate der Formel $M-(Q)_n-R^4$ in Betracht, in der M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, Tetrahydrofuran, Dioxan oder Anisol, Ketone wie Aceton, Butanon oder Cyclohexanon, Amide wie Dimethylformamid oder Phosphorsäure-hexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuss einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden.

Die Reaktionstemperatur liegt gewöhnlich zwischen −50° und +250°, vorzugsweise zwischen −20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure der Formel V mit einem Alkohol oder Phenol der Formel VI in der Regel in Gegenwart einer starken Säure, beispielsweise einer Mineralsäure wie Salzsäure oder Schwefelsäure, umgesetzt.

Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind.

Eine weitere bevorzugte Ausführungsform des Verfahrens nach der Erfindung besteht darin, dass man die zu veresternde Hydroxyverbindung der Formel VI zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder Dimethylformamid versetzt, zweckmässig bei Temperaturen zwischen etwa −25° und +20°.

Die Ausgangsstoffe der Formeln II bis VI sowie ihre reaktionsfähigen Derivate sind zum Teil bekannt, zum Teil können sie in Analogie zu bekannten Verbindungen aus literaturbekannten Stoffen hergestellt werden.

So sind die Amine der Formel II beispielsweise durch Nitrierung von Biphenylderivaten (entsprechend Formel I, aber X = H) und anschliessende Reduktion erhältlich, die Ausgangsstoffe der Formeln III und V analog aus den unsubstituierten Verbindungen (entsprechend Formel III und V, aber X = H) durch Nitrierung, Reduktion zur Aminoverbindung, Diazotierung und Schiemann- bzw. Sandmeyer-Reaktion.

Die erfindungsgemässen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder -cyclohexylester, Phenylcyclohexane, Cyclohexylbiphenyle, Cyclohexylcyclohexane, Cyclohexylnaphthaline, 1,4-Biscyclohexylbenzole, 4,4'-Bis-cyclohexylbiphenyle, Phenyl- oder Cyclohexyl-pyrimidine, Phenyl- oder Cyclohexyldioxane, gegebenenfalls halogenierten Stilbene, Benzylphenylether, Tolane und substituierten Zimtsäuren. Die wichtigsten als Bestandteile derartiger flüssigkristalliner Dielektrika in Frage kommenden Verbindungen lassen sich durch die Formel VII charakterisieren

$$R^5-A-G-E-R^6 \qquad VII$$

worin A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | −CH=CH− | −N(O)=N− |
| | −CH=CL− | −CH=N(O)− |
| | −CH≡C− | $CH_2-CH_2-$ |
| | −CO−O− | $-CH_2-O-$ |
| | −CO−S− | $-CH_2-S-$ |
| | −CH=N− | −COO−Ph−COO− |
| | | oder eine C−C-Einfachbindung, |

L Halogen, vorzugsweise Chlor, oder CN, und $R^5$ und $R^6$ Alkyl, Alkoxy, Alkanoyloxy oder Alkoxycarbonyloxy mit bis zu 18, vorzugsweise bis zu 8 Kohlenstoffatomen, einer dieser Reste auch CN, NC, $NO_2$, $CF_3$, F, Cl oder Br bedeuten. Bei den meisten dieser Verbindungen sind $R^5$ und $R^6$ voneinander verschieden, wobei einer dieser Reste meist eine Alkyl- oder Alkoxygruppe ist. Viele solcher Substanzen oder auch Gemische davon sind im Handel erhältlich.

Die erfindungsgemässen Dielektrika enthalten etwa 0,1 bis 30, vorzugsweise 2 bis 25% einer oder mehrerer Verbindungen der Formel I.

Die Herstellung der erfindungsgemässen Dielektrika erfolgt in an sich üblicher Weise. In der Regel werden die Komponenten ineinander gelöst, zweckmässig bei erhöhter Temperatur. Wenn dabei eine Temperatur oberhalb des Klärpunkts des Hauptbestandteils gewählt wird, kann die Vollständigkeit des Lösungsvorgangs besonders leicht beobachtet werden.

Durch geeignete Zusätze können die flüssigkristallinen Dielektrika nach der Erfindung so modifiziert werden, dass sie in allen bisher bekannt gewordenen Arten von Flüssigkristallanzeigeelementen verwendet werden können.

Derartige Zusätze sind dem Fachmann bekannt

und in der Literatur ausführlich beschrieben. Beispielsweise können dichroitische Farbstoffe oder Substanzen zur Veränderung der dielektrischen Anisotropie, der Viskosität, der Leitfähigkeit und/oder der Orientierung der nematischen Phasen zugesetzt werden. Derartige Substanzen sind z.B. in den DE-OS Nrn. 2209127, 2240864, 2321632, 2338281, 2450088, 2637430, 2853728 und 2902177 beschrieben.

Die folgenden Beispiele sollen die Erfindung erlautern, ohne sie zu begrenzen. In den Beispielen bedeuten F. den Schmelzpunkt und K. den Klärpunkt einer flüssigkristallinen Substanz. Vor- und nachstehend bedeuten Prozentangaben Gewichtsprozent; alle Temperaturen sind in Grad Celsius angegeben.

*Beispiel 1:*

Zu einer Suspension von 36,7 g 4'-Acetyl-2-amino-4-(trans-4-pentylcyclohexyl)biphenyl [F. 118-123°; erhältlich durch Nitrieren von 4'-Acetyl-4-(trans-4-pentylcyclohexyl)biphenyl und anschliessende Hydrierung der erhaltenen rohen 2-Nitroverbindung an Raney-Nickel in Tetrahydrofuran] in 180 ml Dioxan tropft man bei 15-20° unter Rühren 180 ml 35%ige wässerige Tetrafluorborsäurelösung und dann bei 0° eine Lösung von 7,2 g $NaNO_2$ in 25 ml Wasser. Man rührt 6 Stunden nach, wobei man die Temperatur auf 15° steigen lässt. Nach üblicher Aufarbeitung (Extraktion mit Toluol, Filtration über Kieselgel) erhält man 4'-Acetyl-2-fluor-4-(trans-4-pentylcyclohexyl)biphenyl, F. 81°, K. 170°.

*Beispiele 2 bis 49:*

Analog Beispiel 1 erhält man aus den entsprechenden Aminoverbindungen:

2. 4'-Acetyl-2-fluor-4-methylbiphenyl.
3. 4'-Acetyl-2-fluor-4-ethylbiphenyl.
4. 4'-Acetyl-2-fluor-4-propylbiphenyl.
5. 4'-Acetyl-2-fluor-4-butylbiphenyl.
6. 4'-Acetyl-2-fluor-4-pentylbiphenyl.
7. 4'-Acetyl-2-fluor-4-hexylbiphenyl.
8. 4'-Acetyl-2-fluor-4-heptylbiphenyl.
9. 4'-Acetyl-2-fluor-4-octylbiphenyl.
10. 4'-Acetyl-2-fluor-4-nonylbiphenyl.
11. 4'-Acetyl-2-fluor-4-decylbiphenyl.
12. 4'-Acetyl-2-fluor-4-methoxybiphenyl.
13. 4'-Acetyl-2-fluor-4-ethoxybiphenyl.
14. 4'-Acetyl-2-fluor-4-propyloxybiphenyl.
15. 4'-Acetyl-2-fluor-4-butyloxybiphenyl.
16. 4'-Acetyl-2-fluor-4-pentyloxybiphenyl.
17. 4'-Acetyl-2-fluor-4-hexyloxybiphenyl.
18. 4'-Acetyl-2-fluor-4-heptyloxybiphenyl.
19. 4'-Acetyl-2-fluor-4-octyloxybiphenyl.
20. 4'-Acetyl-2-fluor-4-nonyloxybiphenyl.
21. 4'-Acetyl-2-fluor-4-decyloxybiphenyl.
22. 4'-Acetyl-2-fluor-4-(trans-4-methylcyclohexyl)biphenyl.
23. 4'-Acetyl-2-fluor-4-(trans-4-ethylcyclohexyl)biphenyl.
24. 4'-Acetyl-2-fluor-4-(trans-4-propylcyclohexyl)biphenyl.
25. 4'-Acetyl-2-fluor-4-(trans-4-butylcyclohexyl)biphenyl.
26. 4'-Acetyl-2-fluor-4-(trans-4-hexylcyclohexyl)biphenyl.
27. 4'-Acetyl-2-fluor-4-(trans-4-heptylcyclohexyl)biphenyl.
28. 4'-Acetyl-2-fluor-4-(trans-4-octylcyclohexyl)biphenyl.
29. 4'-Acetyl-2-fluor-4-(trans-4-nonylcyclohexyl)biphenyl.
30. 4'-Acetyl-2-fluor-4-(trans-4-decylcyclohexyl)biphenyl.
31. 4'-Acetyl-2-fluor-4-(trans-4-methoxycyclohexyl)biphenyl.
32. 4'-Acetyl-2-fluor-4-(trans-4-ethoxycyclohexyl)biphenyl.
33. 4'-Acetyl-2-fluor-4-(trans-4-propyloxycyclohexyl)biphenyl.
34. 4'-Acetyl-2-fluor-4-(trans-4-butyloxycyclohexyl)biphenyl.
35. 4'-Acetyl-2-fluor-4-(trans-4-pentyloxycyclohexyl)biphenyl.
36. 4'-Acetyl-2-fluor-4-(trans-4-hexyloxycyclohexyl)biphenyl.
37. 4'-Acetyl-2-fluor-4-(trans-4-heptyloxycyclohexyl)biphenyl.
38. 4'-Acetyl-2-fluor-4-(trans-4-octyloxycyclohexyl)biphenyl.
39. 4'-Acetyl-2-fluor-4-(trans-4-nonyloxycyclohexyl)biphenyl.
40. 4'-Acetyl-2-fluor-4-(trans-4-decyloxycyclohexyl)biphenyl.
41. 2-Fluor-4'-propionyl-4-propylbiphenyl.
42. 2-Fluor-4'-propionyl-4-butylbiphenyl.
43. 2-Fluor-4'-propionyl-4-pentylbiphenyl.
44. 2-Fluor-4'-propionyl-4-methoxybiphenyl.
45. 2-Fluor-4'-pripionyl-(trans-4-propylcyclohexyl)biphenyl.
46. 2-Fluor-4'-propionyl-(trans-4-pentylcyclohexyl)biphenyl.
47. 4'-Butyryl-2-fluor-4-(trans-4-propylcyclohexyl)biphenyl.
48. 4'-Decanoyl-2-fluor-4-(trans-4-pentylcyclohexyl)biphenyl.
49. 4'-Acetyl-3-fluor-4-(trans-4-pentylcyclohexyl)biphenyl.

*Beispiel 50:*

Zu einer Suspension von 36,7 g 4'-Acetyl-2-amino-4-(trans-4-pentylcyclohexyl)biphenyl in 150 ml 15%iger wässeriger Salzsäure gibt man bei −5° innerhalb 0,5 Stunden eine Lösung von 7,2 g $NaNO_2$ in 25 ml Wasser. Die so erhaltene Lösung wird bei 0-5° innerhalb 1 Stunde zu einer Cu(I)-salzlösung hinzugetropft, die aus 25 g $CuSO_4 \cdot 5H_2O$, 8,8 g NaCl, 6,3 g $Na_2SO_3$, 100 ml Wasser und 40 ml 32%iger Salzsäure hergestellt worden ist. Nach halbstündigem Erhitzen auf 40° wird abgekühlt, mit $CH_2Cl_2$ extrahiert und wie üblich aufgearbeitet. Man erhält 4'-Acetyl-2-chlor-4-(trans-4-pentylcyclohexyl)biphenyl.

*Beispiel 51 bis 55:*

Analog Beispiel 50 erhält man aus den entsprechenden Aminoverbindungen:

51. 2-Chlor-4'-acetyl-4-propylbiphenyl.
52. 2-Chlor-4'-acetyl-4-butylbiphenyl.

53. 2-Chlor-4'-acetyl-4-pentylbiphenyl.
54. 2-Chlor-4'-acetyl-4-(trans-4-propylcyclohexyl)biphenyl.
55. 2-Chlor-4'-acetyl-4-(trans-4-pentylcyclohexyl)biphenyl.

*Beispiel 56:*

Man arbeitet analog Beispiel 50, verwendet jedoch HBr statt HCl und NaBr statt NaCl und erhält 4'-Acetyl-2-brom-4-(trans-4-pentylcyclohexyl)-biphenyl.

*Beispiel 57:*

Zu einem Gemisch aus 32,4 g 2-Fluor-4-(trans-4-pentylcyclohexyl)biphenyl und 200 ml $CS_2$ gibt man unter Rühren bei 20° 27 g $AlCl_3$, tropft dann unter Rühren und Kochen 10 g Acetanhydrid hinzu und kocht noch eine Stunde. Man kühlt ab, filtriert, zersetzt durch Eintragen in Salzsäure/Eis und erhält 4'-Acetyl-2-fluor-4-(trans-4-pentylcyclohexyl)biphenyl, F. 81°, K. 170°.

Analog sind die in den Beispielen 2 bis 56 genannten Verbindungen erhältlich.

*Beispiel 58:*

Man kocht 28,6 g 2'-Fluor-4'-pentylbiphenyl-4-carbonsäure (erhältlich durch Nitrierung von 4'-Pentylbiphenyl-4-carbonsäure zum 2'-Nitroderivat, Reduktion zur 2'-Aminoverbindung und Schiemann-Reaktion) 1 Stunde mit 24 g $SOCl_2$, dampft ein, löst das erhaltene rohe Säurechlorid in 150 ml Toluol, versetzt mit 7,9 g Pyridin und 6 g Propanol und kocht 2 Stunden. Das Gemisch wird gekühlt, mit Wasser gewaschen, die organische Phase wird über $Na_2SO_4$ getrocknet und eingedampft. Man erhält 2'-Fluor-4'-pentylbiphenyl-4-carbonsäurepropylester.

59. 2'-Fluor-4'-propylbiphenyl-4-carbonsäuremethylester.
60. 2'-Fluor-4'-propylbiphenyl-4-carbonsäureethylester.
61. 2'-Fluor-4'-propylbiphenyl-4-carbonsäurepropylester.
62. 2'-Fluor-4'-propylbiphenyl-4-carbonsäurebutylester.
63. 2'-Fluor-4'-propylbiphenyl-4-carbonsäurepentylester.
64. 2'-Fluor-4'-propylbiphenyl-4-carbonsäurehexylester.
65. 2'-Fluor-4'-propylbiphenyl-4-carbonsäureheptylester.
66. 2'-Fluor-4'-propylbiphenyl-4-carbonsäureoctylester.
67. 2'-Fluor-4'-propylbiphenyl-4-carbonsäure(1-methylheptylester).
68. 2'-Fluor-4'-propylbiphenyl-4-carbonsäurenonylester.
69. 2'-Fluor-4'-propylbiphenyl-4-carbonsäuredecylester.
70. 2'-Fluor-4'-propylbiphenyl-4-carbonsäure(trans-4-propylcyclohexylester).
71. 2'-Fluor-4'-propylbiphenyl-4-carbonsäure(trans-4-butylcyclohexylester).
72. 2'-Fluor-4'-propylbiphenyl-4-carbonsäure(trans-4-pentylcyclohexylester).
73. 2'-Fluor-4'-propylbiphenyl-4-carbonsäure(p-propylphenylester).
74. 2'-Fluor-4'-propylbiphenyl-4-carbonsäure(p-butylphenylester).
75. 2'-Fluor-4'-propylbiphenyl-4-carbonsäure(p-pentylphenylester).
76. 2'-Fluor-4'-propylbiphenyl-4-carbonsäure(p-methoxyphenylester).
77. 2'-Fluor-4'-propylbiphenyl-4-carbonsäure(p-ethoxyphenylester).
78. 4'-Butyl-2'-fluorbiphenyl-4-carbonsäureethylester.
79. 4'-Butyl-2'-fluorbiphenyl-4-carbonsäurepropylester.
80. 4'-Butyl-2'-fluorbiphenyl-4-carbonsäurebutylester.
81. 4'-Butyl-2'-fluorbiphenyl-4-carbonsäurepentylester.
82. 4'-Butyl-2'-fluorbiphenyl-4-carbonsäurehexylester.
83. 4'-Butyl-2'-fluorbiphenyl-4-carbonsäure(1-methylheptylester).
84. 4'-Butyl-2'-fluorbiphenyl-4-carbonsäure(trans-4-propylcyclohexylester).
85. 4'-Butyl-2'-fluorbiphenyl-4-carbonsäure(trans-4-pentylcyclohexylester).
86. 4'-Butyl-2'-fluorbiphenyl-4-carbonsäure(p-propylphenylester).
87. 4'-Butyl-2'-fluorbiphenyl-4-carbonsäure(p-pentylphenylester).
88. 4'-Butyl-2'-fluorbiphenyl-4-carbonsäure(p-methoxyphenylester).
89. 4'-Butyl-2'-fluorbiphenyl-4-carbonsäure(p-ethoxyphenylester).
90. 2'-Fluor-4'-pentylbiphenyl-4-carbonsäureethylester.
91. 2'-Fluor-4'-pentylbiphenyl-4-carbonsäurebutylester.
92. 2'-Fluor-4'-pentylbiphenyl-4-carbonsäurepentylester.
93. 2'-Fluor-4'-pentylbiphenyl-4-carbonsäurehexylester.
94. 2'-Fluor-4'-pentylbiphenyl-4-carbonsäure(1-methylheptylester).
95. 2'-Fluor-4'-pentylbiphenyl-4-carbonsäure(trans-4-propylcyclohexylester).
96. 2'-Fluor-4'-pentylbiphenyl-4-carbonsäure(trans-4-pentylcyclohexylester).
97. 2'-Fluor-4'-pentylbiphenyl-4-carbonsäure(p-propylphenylester).
98. 2'-Fluor-4'-pentylbiphenyl-4-carbonsäure(p-pentylphenylester).
99. 2'-Fluor-4'-pentylbiphenyl-4-carbonsäure(p-methoxyphenylester).
100. 2'-Fluor-4'-pentylbiphenyl-4-carbonsäure(p-ethoxyphenylester).
101. 2'-Fluor-4'-(trans-4-propylcyclohexyl)biphenyl-4-carbonsäureethylester.
102. 2'-Fluor-4'-(trans-4-propylcyclohexyl)biphenyl-4-carbonsäurepropylester.
103. 2'-Fluor-4'-(trans-4-propylcyclohexyl)biphenyl-4-carbonsäurebutylester.
104. 2'-Fluor-4'-(trans-4-propylcyclohexyl)biphenyl-4-carbonsäurepentylester.

105. 2'-Fluor-4'-(trans-4-propylcyclohexyl)biphenyl-4-carbonsäurehexylester.
106. 2'-Fluor-4'-(trans-4-propylcyclohexyl)biphenyl-4-carbonsäure-1-methylheptylester).
107. 2'-Fluor-4'-(trans-4-propylcyclohexyl)biphenyl-4-carbonsaure(trans-4-propylcyclohexylester).
108. 2'-Fluor-4'-(trans-4-propylcyclohexyl)biphenyl-4-carbonsäure(trans-4-pentylcyclohexylester).
109. 2'-Fluor-4'-(trans-4-propylcyclohexyl)biphenyl-4-carbonsäure(p-propylphenylester).
110. 2'-Fluor-4'-(trans-4-propylcyclohexyl)biphenyl-4-carbonsäure(p-pentylphenylester).
111. 2'-Fluor-4'-(trans-4-propylcyclohexyl)biphenyl-4-carbonsäure(p-methoxyphenylester).
112. 2'-Fluor-4'-(trans-4-propylcyclohexyl)biphenyl-4-carbonsäure(p-ethoxyphenylester).
113. 4'-(trans-4-Butylcyclohexyl)-2'-fluorbiphenyl-4-carbonsäureethylester.
114. 4'-(trans-4-Butylcyclohexyl)-2'-fluorbiphenyl-4-carbonsäurepropylester.
115. 4'-(trans-4-Butylcyclohexyl)-2'-fluorbiphenyl-4-carbonsäurebutylester.
116. 4'-(trans-4-Butylcyclohexyl)-2'-fluorbiphenyl-4-carbonsäurepentylester.
117. 4'-(trans-4-Butylcyclohexyl)-2'-fluorbiphenyl-4-carbonsäurehexylester.
118. 4'-(trans-4-Butylcyclohexyl)-2'-fluorbiphenyl-4-carbonsäure-1-(1-methylheptylester).
119. 4'-(trans-4-Butylcyclohexyl)-2'-fluorbiphenyl-4-carbonsäure(trans-4-propylcyclohexylester).
120. 4'-(trans-4-Butylcyclohexyl)-2'-fluorbiphenyl-4-carbonsäure(trans-4-pentylcyclohexylester).
121. 4'-(trans-4-Butylcyclohexyl)-2'-fluorbiphenyl-4-carbonsäure(p-propylphenylester).
122. 4'-(trans-4-Butylcyclohexyl)-2'-fluorbiphenyl-4-carbonsäure(p-pentylphenylester).
123. 4'-(trans-4-Butylcyclohexyl)-2'-fluorbiphenyl-4-carbonsäure(p-methoxyphenylester).
124. 4'-(trans-4-Butylcyclohexyl)-2'-fluorbiphenyl-4-carbonsäure(p-ethoxyphenylester).
125. 2'-Fluor-4'-(trans-4-pentylcyclohexyl)biphenyl-4-carbonsäureethylester, F. 53,5°, K. 153,4°.
126. 2'-Fluor-4'-(trans-4-pentylcyclohexyl)biphenyl-4-carbonsäurepropylester.
127. 2'-Fluor-4'-(trans-4-pentylcyclohexyl)biphenyl-4-carbonsäurebutylester, F. 52°, K. 120,3°.
128. 2'-Fluor-4'-(trans-4-pentylcyclohexyl)biphenyl-4-carbonsäurepentylester.
129. 2'-Fluor-4'-(trans-4-pentylcyclohexyl)biphenyl-4-carbonsäurehexylester.
130. 2'-Fluor-4'-(trans-4-pentylcyclohexyl)biphenyl-4-carbonsäure(1-methylheptylester).
131. 2'-Fluor-4'-(trans-4-pentylcyclohexyl)biphenyl-4-carbonsäure(trans-4-propylcyclohexylester).
132. 2'-Fluor-4'-(trans-4-pentylcyclohexyl)biphenyl-4-carbonsäure(trans-4-pentylcyclohexylester).
133. 2'-Fluor-4'-(trans-4-pentylcyclohexyl)biphenyl-4-carbonsäure(p-propylphenylester).
134. 2'-Fluor-4'-(trans-4-pentylcyclohexyl)biphenyl-4-carbonsäure(p-pentylphenylester).
135. 2'-Fluor-4'-(trans-4-pentylcyclohexyl)biphenyl-4-carbonsäure(p-methoxyphenylester).
136. 2'-Fluor-4'-(trans-4-pentylcyclohexyl)biphenyl-4-carbonsäure(p-ethoxyphenylester).

Es folgen Beispiele für Dielektrika mit einem Gehalt an mindestens einer Verbindung der Formel I:

*Beispiel A:*

Ein Gemisch aus

| | |
|---|---|
| 28% | p-(trans-4-Pentylcyclohexyl)benzonitril |
| 11% | 2-p-Cyanphenyl-5-pentyl-1,3-dioxan |
| 32% | 4-Pentyl-4'-cyanbiphenyl |
| 9% | 4-(trans-4-Pentylcyclohexyl)-4'-cyanbiphenyl |
| 20% | 4'-Acetyl-2-fluor-4-(trans-4-pentylcyclohexyl)biphenyl |

zeigt F. −11°, K. 80°.

*Beispiel B:*

Ein Gemisch aus

| | |
|---|---|
| 18% | p-(trans-4-Propylcyclohexyl)benzonitril |
| 26% | p-(trans-4-Pentylcyclohexyl)benzonitril |
| 13% | trans-4-Propylcyclohexancarbonsäure-(p-ethoxyphenylester) |
| 12% | trans-4-Butylcyclohexancarbonsäure-(p-ethoxyphenylester) |
| 14% | trans-4-Pentylcyclohexancarbonsäure-(p-methoxyphenylester) |
| 17% | 4'-Acetyl-2-fluor-4-(trans-4-pentylcyclohexyl)biphenyl |

zeigt F. −14°, K. 77°.

*Beispiel C:*

Ein Gemisch aus

| | |
|---|---|
| 13% | p-(trans-4-Ethylcyclohexyl)benzonitril |
| 18% | p-(trans-4-Propylcyclohexyl)benzonitril |
| 13% | p-(trans-4-Butylcyclohexyl)benzonitril |
| 25% | p-(trans-4-Pentylcyclohexyl)benzonitril |
| 15% | p-(trans-4-Heptylcyclohexyl)benzonitril |
| 16% | 4'-Acetyl-2-fluor-4-(trans-4-pentylcyclohexyl)biphenyl |

zeigt F. −17°, K. 58°.

## Patentansprüche

1. Halogenbiphenylderivate der Formel I

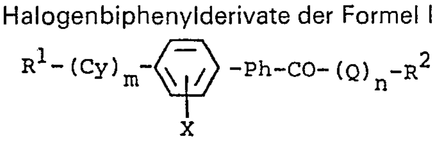

I

worin
$R^1$ und $R^2$ jeweils Alkyl oder Alkoxy mit jeweils 1-10 C-Atomen,
Cy 1,4-Cyclohexylen,
Ph 1,4-Phenylen,
Q –O–Cy– oder –O–Ph–
m und n jeweils 0 oder 1 und
X F, Cl oder Br
bedeuten.

2. 4'-Acetyl-2-fluor-4-(4-trans-n-pentylcyclohexyl)biphenyl.

3. Verfahren zur Herstellung von Halogenbiphenylderivaten der Formel I

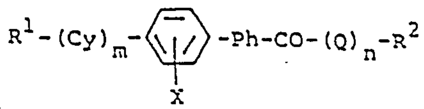 I

worin
$R^1$ und $R^2$ jeweils Alkyl oder Alkoxy mit jeweils 1-10 C-Atomen,
Cy 1,4-Cyclohexylen,
Ph 1,4-Phenylen,
Q –O–Cy– oder –O–Ph–
m und n jeweils 0 oder 1 und
X F, Cl oder Br
bedeuten,
dadurch gekennzeichnet, dass man ein Aminobiphenylderivat der Formel II

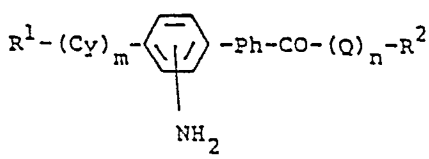 II

worin
$R^1$, $R^2$, Cy, Ph, Q, m und n die bei Formel I angegebene Bedeutung haben diazotiert und anschliessend die Diazoniumgruppe nach bekannten Methoden durch ein X-Atom ersetzt oder dass man zur Herstellung einer Verbindung der Formel I ($R^2$ = Alkyl, n = 0) ein Halogenbiphenylderivat der Formel III

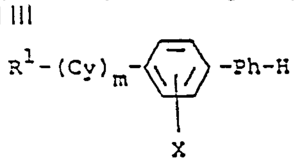 III

worin
$R^1$, Cy, Ph, m und X die bei Formel I angegebene Bedeutung haben mit einer Carbonsäure der Formel IV

$$HOOC-R^3 \qquad IV$$

worin
$R^3$ Alkyl mit 1-10 C-Atomen bedeutet oder mit einem ihrer reaktionsfähigen Derivate in Gegenwart einer Säure oder einer Lewis-Säure umsetzt, oder dass man zur Herstellung einer Verbindung der Formel I ($R^2$ = Alkoxy und/oder n = 1) eine Carbonsäure der Formel V

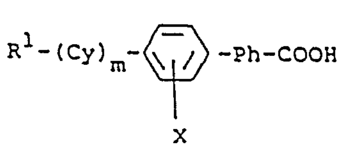 V

worin
$R^1$, Cy, Ph, m und X die bei Formel I angegebene Bedeutung haben, oder eines ihrer reaktionsfähigen Derivate mit einer Hydroxyverbindung der Formel VI

$$H-(Q)_n-R^4 \qquad VI$$

worin
$R^4$ Alkoxy mit 1-10 C-Atomen bedeutet und, falls n = 1, auch Alkyl mit 1-10 C-Atomen bedeuten kann und
Q und n die bei Formel I angegebene Bedeutung haben,
oder einem ihrer reaktionsfähigen Derivate umsetzt.

4. Verwendung der Verbindungen der Formel I nach Anspruch 1 als Komponenten flüssigkristalliner Dielektrika für elektrooptische Anzeigeelemente.

5. Flüssigkristallines Dielektrikum für elektrooptische Anzeigeelemente mit mindestens zwei flüssigkristallinen Komponenten, dadurch gekennzeichnet, dass mindestens eine Komponente eine Verbindung der Formel I nach Anspruch 1 ist.

6. Elektrooptisches Anzeigeelement auf der Basis einer Flüssigkristallzelle, dadurch gekennzeichnet, dass die Flüssigkristallzelle ein Dielektrikum nach Anspruch 5 enthält.

**Claims**

1. Halogenobiphenyl derivatives of the formula I

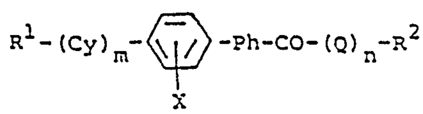 I

wherein $R^1$ and $R^2$ are each alkyl or alkoxy with in each case 1-10 C atoms, Cy is 1,4-cyclohexylene, Ph is 1,4-phenylene, Q is –O–Cy– or –O–Ph–, m and n are each 0 or 1 and X is F, Cl or Br.

2. 4'-Acetyl-2-fluoro-4-(4-trans-n-pentylcyclohexyl)biphenyl.

3. Process for the preparation of halogenobiphenyl derivatives of the formula I

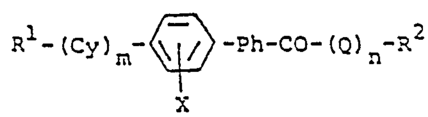 I

wherein $R^1$ and $R^2$ are each alkyl or alkoxy with in each case 1-10 C atoms, Cy is 1,4-cyclohexylene, Ph is 1,4-phenylene, Q is –O–Cy– or –O–Ph–, m and n are each 0 or 1 and X is F, Cl or Br, characterised in that an aminobiphenyl derivative of the formula II

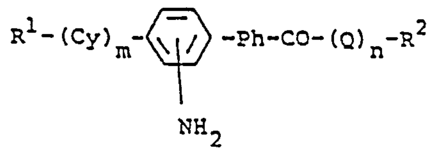 II

wherein $R^1$. $R^2$ Cy, Ph, Q, m and n have the meaning given in the case of formula I, is diazotised and the diazonium group is then replaced by an X atom by known methods, or in that, for the preparation

of a compound of the formula I ($R^2$ is alkyl, n is 0), a halogenobiphenyl derivative of the formula III

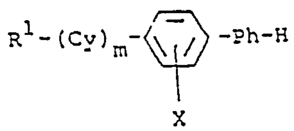 III

wherein $R^1$, Cy, Ph, m and X have the meaning given in the case of formula I, is reacted with a carboxylic acid of the formula IV

$$HOOC-R^3 \qquad IV$$

wherein $R^3$ is alkyl with 1-10 C atoms, or with one of its reactive derivatives, in the presence of an acid or a Lewis acid, or in that, for the preparation of a compound for the formula I ($R^2$ is alkoxy and/or n is 1), a carboxylic acid of the formula V

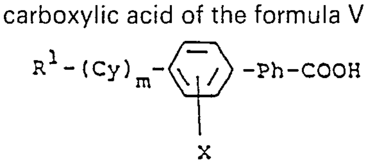 V

wherein $R^1$, Cy, Ph, m and X have the meaning given in the case of formula I, or one of its reactive derivatives, is reacted with a hydroxy compound of the formula VI

$$H-(Q)_n-R^4 \qquad VI$$

wherein $R^4$ is alkoxy with 1-10 C atoms, or, if n is 1, can also be alkyl with 1-10 C atoms, and Q and n have the meaning given in the case of formula I, or one of its reactive derivatives.

4. Use of the compounds of the formula I according to Claim 1 as components of liquide crystal dielectrics for electrooptical display elements.

5. Liquid crystal dielectric for electrooptical display elements with at least two crystal components, characterised in that at least one component is a compound of the formula I according to Claim 1.

6. Electrooptical display element based on a liquid crystal cell, characterised in that the liquid crystal cell contains a dielectric according to Claim 5.

## Revendications

1. Dérivés d'halogènebiphényle de formule I

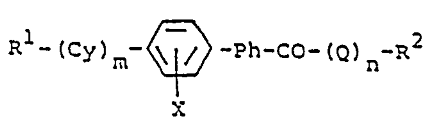 I

où

$R^1$ et $R^2$ représentent chacun un alcoxy avec à chaque fois de 1 à 10 atomes de carbone,
Cy représente un 1,4-cyclohexylène,
Ph représente un 1,4-phénylène,
Q représente $-O-Cy-$ ou $-O-Ph-$
m et n valent l'un et l'autre 0 ou 1 et
X représente F, Cl ou Br.

2. 4'-Acétyl-2-fluoro-4-(4-trans-n-pentylcyclohexyl)biphényle.

3. Procédé de préparation de dérivés d'halogènebiphényle de formule I

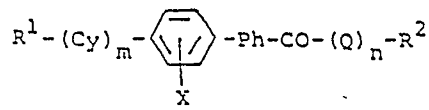 I

où

$R^1$ et $R^2$ représentent chacun un alcoyle ou un alcoxy avec à chaque fois de 1 à 10 atomes de carbone,
Cy représente un 1,4-cyclohexylène,
Ph représente un 1,4-phénylène,
Q représente $-O-Cy-$ ou $-O-Ph-$
m et n valent l'un et l'autre 0 ou 1 et
X représente F, Cl ou Br,

caractérisé en ce qu'on diazote un dérivé d'aminobiphényle de formule II

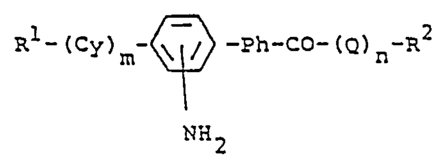 II

où

$R^1$, $R^2$, Cy, Ph, Q, m et n ont la signification donnée pour la formule I, puis en ce qu'on remplace le groupe diazonium selon des procédés connus par un atome de X ou en ce que pour préparer un composé de formule I ($R^2$ = alcoyle, n = 0) on fait réagir un dérivé d'halogènebiphényle de formule III

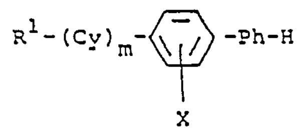 III

où

$R^1$, Cy, Ph, m et X ont la signification donnée pour la formule I avec un acide carboxylique de formule IV

$$HOOC-R^3 \qquad IV$$

où

$R^3$ représente un alcoyle en C1 à C10,
ou avec un de ses dérivés réactifs en présence d'un acide ou d'un acide de Lewis,
ou en ce que pour préparer un composé de formule I ($R^2$ = alcoxy et/ou n = 1) on fait réagir un acide carboxylique de formule V

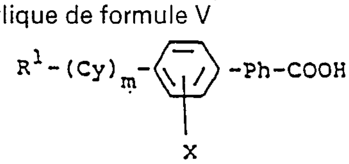 V

où

$R^1$, Cy, Ph, m et X ont la signification donnée pour la formule I,
ou un de ses dérivés réactifs,
avec un composé hydroxy de formule VI

$$H-(Q)_n-R^4 \qquad VI$$

où

$R^4$ représente un alcoxy en C1 à C10 et peut également, si n = 1, représenter un alcoyle en C1 à C10 et
Q et n ont la signification donnée pour la formule I, ou avec un de ses dérivés réactifs.

4. Application des composés de formule I selon la revendication 1 comme composants de diélectriques à cristaux liquides pour éléments d'affichage électro-optiques.

5. Diélectrique à cristaux liquides pour éléments d'affichage électro-optiques avec au moins deux composants à cristaux liquides, caractérisé en ce qu'au moins un composant est un composé de formule I selon la revendication 1.

6. Elément d'affichage électro-optique à base d'une cellule à cristaux liquides, caractérisé en ce que la cellule à cristaux liquides contient un diélectrique selon la revendication 5.